# EUROPEAN PATENT APPLICATION

(11) **EP 4 782 029 A1**
(43) Date of publication of application: **29.07.2026**
(21) Application number: 23952823.5
(22) Date of filing: 25.10.2023
(51) Int. Cl.: A61M 5/142, A61M 5/145

(54) **CLOSED-LOOP ARTIFICIAL PANCREAS SYSTEM**

(30) Priority: 19.09.2023 WO PCT/CN2023/119735
(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2023/126418
(87) International publication number: WO 2025/060174

(57) **Abstract**

The invention discloses a closed-loop artificial pancreas system, comprising: a detection mechanism, an infusion mechanism, and a program mechanism, at least based on the blood glucose value detected by the detection mechanism, the program mechanism generates an insulin infusion instruction and controls the infusion mechanism to perform the infusion, the system adopts an infusion strategy consisting of pre-infusion and supplemental infusion, and an amount of insulin for pre-infusion is related to an estimated meal size at a time of pre-infusion, an amount of insulin for supplemental infusion is related to an estimated meal size at a time of supplemental infusion. There is no need to explicitly determine the size of the meal, and the system determines the amount of insulin to be infused based on the size of the predicted meal, on the one hand, relieving the pressure to accurately determine the size of the meal, and at the same time, taking into account the dynamic impact on blood glucose levels after the meal and the interactive effects of the bolus itself. The system adopts the infusion strategy consisting of pre-infusion and supplemental infusion, maintaining the patient's glucose concentration at a desirable level.

## Description

### TECHNICAL FIELD

The present invention mainly relates to the field of medical device, and in particular to a closed-loop artificial pancreas system.

### BACKGROUND

The pancreas of healthy people can automatically secrete the required insulin/glucagon according to the glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods of continuously monitoring blood glucose are based on in vivo glucose monitoring devices, which use disposable transdermal sensors inserted into the skin to measure glucose concentrations in interstitial fluids and send the blood glucose data through a transmitter to the remote device in real-time for the user to view. This monitoring method is called Continuous Glucose Monitoring (CGM). Based on the blood glucose value detected by the CGM, the insulin pump automatically adjusts the amount of insulin required by the patient and infuses it subcutaneously, thus constituting a closed-loop artificial pancreas.

Although current control system of closed-loop artificial pancreases allow CGM and insulin pump to monitor and adjust a patient's blood glucose levels in a substantially continuous and autonomous manner, since the control system is not yet able to accurately recognize the type of meal, the size of the meal, etc. As a result, insulin infusions are typically initiated manually before or during eating, such as, a meal bolus or a corrected bolus, is delivered to prevent fluctuations in a patient's blood glucose levels which may arise from carbohydrate intake or system response delays. In general, meal bolus or corrected bolus infused after eating are a single, one-time infusion. This approach fails to dynamically account for postprandial blood glucose fluctuations and the interactive effects of the bolus itself. It also does not consider the residual influence of a previous meal when calculating the insulin dose for the next meal, often leading to suboptimal glycemic control for the patient.

Therefore, in the prior art, there is an urgent need for a closed-loop artificial pancreas system that can dynamically account for postprandial blood glucose fluctuations and the interactive effects of the bolus itself .

### BRIEF SUMMARY OF THE INVENTION

The embodiment of the present invention discloses a closed-loop artificial pancreas system, comprising: a detection mechanism, an infusion mechanism, and a program mechanism, at least based on the blood glucose value detected by the detection mechanism, the program mechanism generates an insulin infusion instruction and controls the infusion mechanism to perform the infusion, the system adopts an infusion strategy consisting of pre-infusion and supplemental infusion, and an amount of insulin for pre-infusion is related to an estimated meal size at a time of pre-infusion, an amount of insulin for supplemental infusion is related to an estimated meal size at a time of supplemental infusion. There is no need to explicitly determine the size of the meal, and the system determines the amount of insulin to be infused based on the size of the predicted meal, on the one hand, relieving the pressure to accurately determine the size of the meal, and at the same time, taking into account the dynamic impact on blood glucose levels after the meal and the interactive effects of the bolus itself. The system adopts the infusion strategy consisting of pre-infusion and supplemental infusion, maintaining the patient's glucose concentration at a desirable level.

The invention discloses a personalized closed-loop artificial pancreas system, comprising: a detection mechanism, configured to continuously detect a blood glucose value of a patient; an infusion mechanism, configured to infuse insulin into the patient; a program mechanism, configured to control the detection mechanism and the infusion mechanism, and at least based on the blood glucose value detected by the detection mechanism, the program mechanism generates an insulin infusion instruction and controls the infusion mechanism to perform the infusion, the system adopts an infusion strategy consisting of pre-infusion and supplemental infusion, and an amount of insulin for pre-infusion is related to an estimated meal size at a time of pre-infusion, an amount of insulin for supplemental infusion is related to an estimated meal size at a time of supplemental infusion.

According to one aspect of the present invention, the estimated meal size at the time of pre-infusion or supplemental infusion comprises different levels.

According to one aspect of the present invention, the system determines whether to adjust the level of a next pre-infusion based on occurrence or absence of hyperglycemia and hypoglycemia within a predetermined time period after the pre-infusion.

According to one aspect of the present invention, the level of next pre-infusion is not changed when the meal size of pre-infusion is at the highest level and hyperglycemia occurs within a predetermined time period after the pre-infusion, or when the meal size of pre-infusion is at the lowest level and hypoglycemia occurs within a predetermined time period after the pre-infusion.

According to one aspect of the present invention, the system confirms whether to perform the supplemental infusion at least based on a blood glucose value after the pre-infusion has been completed for a predetermined period of time.

According to one aspect of the present invention, the system determines whether to change the level of a next supplemental infusion based on occurrence or absence of hyperglycemia and hypoglycemia within a predetermined time period after the supplemental infusion.

According to one aspect of the present invention, the next supplemental infusion level is not changed when the meal size of supplemental infusion is at the highest level and hyperglycemia occurs within a predetermined time period after the supplemental infusion, or when the meal size of supplemental infusion is at the lowest level and hypoglycemia occurs within a predetermined time period after the supplemental infusion.

According to one aspect of the present invention, the amount of insulin for pre-infusion is related to an actual blood glucose value or its rate of change or IOB at a time of pre-infusion.

According to one aspect of the present invention, the amount of insulin for supplemental infusion is related to an actual blood glucose value or its rate of change or IOB at a time of supplemental infusion.

According to one aspect of the present invention, the program mechanism performs different meal mode function, the meal mode comprises a high carbohydrate mode and a normal carbohydrate mode.

According to one aspect of the present invention, an amount of insulin to be infused during the supplemental infusion is at least related to an actual blood glucose value or rate of change of blood glucose at a time the supplemental infusion is performed, as well as an estimated meal size.

According to one aspect of the present invention, the default estimated meal size is any of the different meal size levels.

According to one aspect of the present invention, the default estimated meal size is the lowest of the different meal size levels.

According to one aspect of the present invention, the system confirms whether to perform the supplemental infusion at least based on a blood glucose value after the supplemental infusion has been completed for a predetermined period of time.

According to one aspect of the present invention, the estimated meal size for the highest level of the regular mode is not greater than the estimated meal size for the lowest level of the large mode at the time of pre-infusion.

According to one aspect of the present invention, the estimated meal size for the highest level of the regular mode is not greater than the estimated meal size for the lowest level of to the large mode at the time of supplemental infusion.

According to one aspect of the present invention, the different levels of estimated meal size of pre-infusion are independent of that of supplemental infusion each other.

According to one aspect of the present invention, the estimated meal size of pre-infusion is greater than that of supplemental infusion when they are at the same level.

According to one aspect of the present invention, the high carbohydrate mode and/or the normal carbohydrate mode further comprise a small carbohydrate mode, the system performs insulin infusion based on a default estimated meal size in the small meal carbohydrate mode.

Compared with the prior art, the technical solution of the present invention has the following advantages:
In the closed-loop artificial pancreas system disclosed in the present invention, the system comprises a personalized closed-loop artificial pancreas system, comprising: a detection mechanism, an infusion mechanism, and a program mechanism, at least based on the blood glucose value detected by the detection mechanism, the program mechanism generates an insulin infusion instruction and controls the infusion mechanism to perform the infusion, the system adopts an infusion strategy of pre-infusion and supplemental infusion, and an amount of insulin for pre-infusion is related to an estimated meal size at a time of pre-infusion, an amount of insulin for supplemental infusion is related to an estimated meal size at a time of supplemental infusion. There is no need to explicitly determine the size of the meal, and the system determines the amount of insulin to be infused based on the size of the predicted meal, on the one hand, relieving the pressure to accurately determine the size of the meal, and at the same time, taking into account the dynamic impact on blood glucose levels after the meal and the interactive effects of the bolus itself. The system adopts the infusion strategy consisting of pre-infusion and supplemental infusion, maintaining the patient's glucose concentration at a desirable level.

Furthermore, the estimated meal size for pre-infusion or supplemental infusion comprises different levels, the timing and amount of insulin infusion can be adjusted more effectively, resulting in more accurate insulin infusion and improved glycemic control in patients.

Furthermore, the system confirms whether to perform the supplemental infusion at least based on a blood glucose value after the pre-infusion has been completed for a predetermined period of time, determines whether to adjust the level of a next pre-infusion based on occurrence or absence of hyperglycemia and hypoglycemia within a predetermined time period after the pre-infusion, and determines whether to adjust the level of a next supplemental infusion based on occurrence or absence of hyperglycemia and hypoglycemia within a predetermined time period after the supplemental infusion, thus, the patient's blood glucose concentration is always maintained at a stable level.

Furthermore, not only the actual blood glucose value or rate of change of blood glucose, the estimated meal size, but also the IOB in the body at the time of performing the pre-infusion/supplemental infusion are taken into account, so as to make the amount of insulin infused during the pre-infusion and/or supplemental infusion more accurate, and to improve the glycemic control effect of the patients.

Furthermore, the high carbohydrate mode includes at least a large meal mode and a regular meal mode, so that allowing even patients with high carbohydrate intake to select the regular-meal mode. This accommodates situations where such patients consume a relatively small amount of carbohydrates, enabling the system to deliver precise insulin dosing and thereby improve the patient's glycemic control.

Furthermore, the high carbohydrate mode and/or the normal carbohydrate mode also comprise a small carbohydrate mode, when the patient selects the small carbohydrate mode, the system infuses the insulin based on the default estimated meal size, and taking into account the patient's actual blood glucose value or rate of change of blood glucose or IOB in the body at the time when eating a small meal, so that the patient's blood glucose level can be effectively controlled at different times and when eating different amount of carbohydrates.

Furthermore, the next pre-infusion and/or supplemental infusion level is not changed when the meal size of pre-infusion and/or supplemental infusion is at the highest level and hyperglycemia occurs within a predetermined time period after the pre-infusion and/or supplemental infusion, or when the meal size of pre-infusion and/or supplemental infusion is at the lowest level and hypoglycemia occurs within a predetermined time period after the pre-infusion and/or supplemental infusion. Specifically, it sets safe infusion boundaries for insulin delivery, effectively preventing both hyperglycemia and hypoglycemia.

Furthermore, before pre-infusion, if the meal type selected by the patient is changed for the first time, the system carries out pre-infusion or supplemental infusion based on the default estimated meal size, so as to avoid that the meal type has been changed but the next infusion still based on the original meal type, which cause inaccurate infusion and affecting the patient's blood glucose control level; When the patient changes the meal type again, the system carries out pre-infusion or supplemental infusion based on the same levels as the previous pre-infusion and previous supplemental infusion of the same meal type, based on the previous and same meal type and size, so that the insulin infusion during the current meal type is more accurate and the patient's blood glucose level is better controlled.

Furthermore, the default estimated meal size corresponding to the pre-infusion and/or supplemental infusion is the smallest level of the different meal size levels, and the smallest infusion amount for pre-infusion and/or supplemental infusion is used as the initial pre-infusion and/or supplemental infusion, which can effectively prevent hypoglycemia.

### BRIEF DESCRIPTION OF THE DRAWINGS

FIG.1 is a schematic diagram of the module relationship of the general closed-loop artificial pancreas insulin infusion control system according to the embodiment of the present invention.
FIG.2 is a schematic diagram of the integrated continuous glucose monitoring device according to an embodiment of the present invention.
FIG.3 is a schematic diagram of the split continuous glucose monitoring device according to an embodiment of the present invention.
FIG.4a is a schematic diagram of the integrated insulin pump according to an embodiment of the present invention.
FIG.4b is a schematic diagram of the split insulin pump according to an embodiment of the present invention.
FIG. 5a is a schematic diagram of the main interface when the control system is in a first working mode according to an embodiment of the present invention.
FIG. 5b is a schematic diagram of the main interface when the control system is in a second working mode according to an embodiment of the present invention.
FIG. 6a-FIG. 6c are schematic diagrams of different operations for the control system to turn on the insulin pump function according to embodiments of the present invention.
FIG. 7a-FIG. 7c are schematic diagrams of different operations for the control system to turn on the auto mode function according to embodiments of the present invention.
FIG. 8a and FIG. 8b show schematic diagrams of the APP interface before and after turning on the auto mode function of the control system according to an embodiment of the present invention.
FIG. 9a is a schematic diagram of the interface when the high carbohydrate mode is turned on of the system according to embodiments of the present invention.
FIG. 9b is a schematic diagram of the interface when the large meal is selected in the high carbohydrate mode of the system according to embodiments of the present invention.
FIG. 9c is a schematic diagram of the interface when the regular meal is selected in the high carbohydrate mode of the system according to embodiments of the present invention.
FIG. 9d is a schematic diagram of the interface when the normal carbohydrate mode is turned on of the system according to embodiments of the present invention.
FIG. 10 is a schematic diagram of the process of an infusion strategy for pre-infusion and supplemental infusion according to embodiments of the present invention.

### DETAILED DESCRIPTION

As mentioned above, meal bolus or corrected bolus infused after the user has eaten a meal are a single infused bolus, failing to dynamically consider the dynamic effects on blood glucose levels after the meal and t the interactive effects of the bolus itself. It also does not consider the residual influence of a previous meal when calculating the insulin dose for the next meal, often leading to suboptimal glycemic control for the patient, thus a closed-loop artificial pancreas system that can dynamically consider the dynamic effects on blood glucose levels after the meal and the infused bolus is needed.

In order to solve this problem, the present invention provides a closed-loop artificial pancreas system, comprising: a detection mechanism, an infusion mechanism, and a program mechanism, at least based on the blood glucose value detected by the detection mechanism, the program mechanism generates an insulin infusion instruction and controls the infusion mechanism to perform the infusion, the system utilizes an infusion strategy of pre-infusion and supplemental infusion, and an amount of insulin for pre-infusion is related to an estimated meal size at a time of pre-infusion, an amount of insulin for supplemental infusion is related to an estimated meal size at a time of supplemental infusion. There is no need to explicitly determine the size of the meal, and the system determines the amount of insulin to be infused based on the size of the predicted meal, on the one hand, relieving the pressure to accurately determine the size of the meal, and at the same time, taking into account the dynamic impact on blood glucose levels after the meal and the infusion of a bolus, the system adopts the infusion strategy of pre-infusion and supplemental infusion, so as to keep the patient's glucose concentration at a desirable level.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other parts.

The following description of the exemplary embodiments is merely illustrative, and is not intended to be in any way limiting the invention and its application or use. The techniques, methods, and devices that are known to those of ordinary skill in the art may not be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

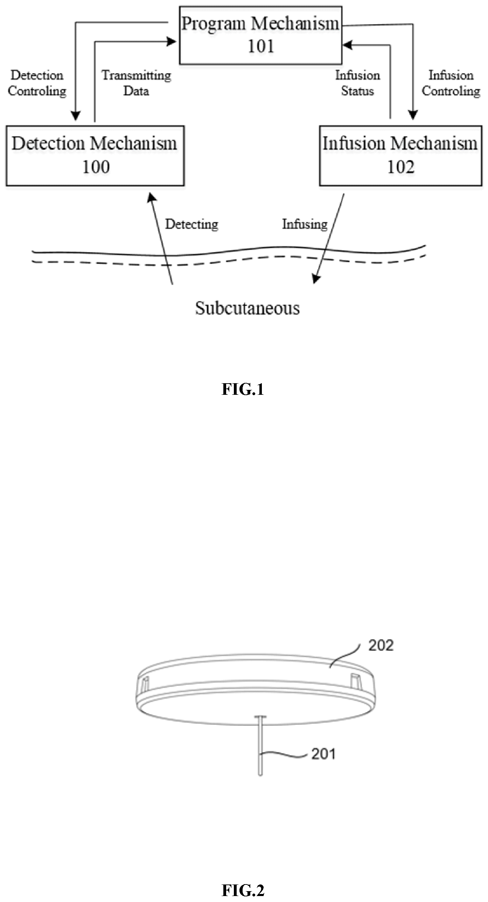

FIG.1 is a schematic diagram of the module relationship of a general closed-loop artificial pancreas insulin infusion control system according to the embodiment of the present invention.

The closed-loop artificial pancreas insulin infusion control system disclosed in the embodiment of the present invention mainly includes a detection mechanism 100, a program mechanism 101, and an infusion mechanism 102.

The detection mechanism 100 is used to continuously detect the user's real-time blood glucose (BG) level. Generally, detection mechanism 100 is a Continuous Glucose Monitoring (CGM) for detecting real-time BG, monitoring BG changes, and sending them to the program mechanism 101. The CGM includes an implantable sensor, the sensor is connected to a transmitter, the transmitter further includes a memory, a processor, a communication interface, and the like, and the transmitter is used for transmitting at least information about the blood glucose data monitored by the CGM, as well as information about an identifier of the CGM, and the like.

The infusion mechanism 102 includes the essential mechanical assemblies and electronic control unit used to infuse insulin, such as reservoir, drive structures, fluid path and infusion needles, power supplies, circuit boards, and so on, and is controlled by program mechanism 101. Generally, the infusion mechanism 102 is an insulin pump, and the electronic control unit includes a memory, a processor, a communication interface, etc. According to the current insulin infusion dose sent by program mechanism 101, infusion mechanism 102 injects the current insulin dose required into the user's body. At the same time, the real-time infusion status of infusion mechanism 102 can also be fed back to program mechanism 101.

Program mechanism 101 is used to control the detection mechanism 100 and the infusion mechanism 102. Therefore, program mechanism 101 is connected to detection mechanism 100 and infusion mechanism 102, respectively. Here, the connection refers to a conventional electrical connection or a wireless connection.

The embodiment of the present invention does not limit the specific positions and connection relationships of the detection mechanism 100, the program mechanism 101 and the infusion mechanism 102, as long as the aforementioned functional conditions can be satisfied.

As in an embodiment of the present invention, the three are electrically connected to form a single part. Therefore, the three mechanisms can be attached on only one position of the user's skin. If the three mechanisms are connected as a whole and attached in only one position, the number of the device on the user skin will be reduced, thereby reducing the interference of more attached devices on user activities. At the same time, it also effectively solves the problem of poor wireless communication between separating devices, further enhancing the user experience.

Another embodiment of the present invention is that the program mechanism 101 and the infusion mechanism 102 are electrically connected to form a single part, while the detection mechanism 100 is separately provided in another part. At this time, the detection mechanism 100 and the program mechanism 101 transmit wireless signals to realize the mutual connection. Therefore, program mechanism 101 and infusion mechanism 102 can be attached to the user's skin position while the detection mechanism 100 is attached to the other position.

Another embodiment of the present invention is that the program mechanism 101 and the detection mechanism 100 are electrically connected, forming a single part, while the infusion mechanism 102 is separately provided in another part. The infusion mechanism 102 and the program mechanism 101 transmit wireless signals to realize the mutual connection. Therefore, program mechanism 101 and the detection mechanism 100 can be attached to the same position of the user's skin while the infusion mechanism 102 is attached to the other position.

Another embodiment of the present invention is that the three are provided in different parts, thus being attached to different positions. Simultaneously, program mechanism 101, detection mechanism 100, and infusion mechanism 102 transmit wireless signals to realize the mutual connection.

Another embodiment of the present invention is that the three are provided in different parts, the detection mechanism 100 and the infusion mechanism 102 are attached to different locations of the user's skin, respectively, while the program mechanism 101 does not have to be attached to the skin, and control the detection mechanism 100 and the infusion mechanism 102 by means of a handheld or portable device. At this time, the program mechanism 101 transmits wireless signals to the detection mechanism 100 and the infusion mechanism 102, respectively, to achieve connection with each other.

The wireless connections described in the foregoing embodiments include, but are not limited to, radio frequency (RF) communications (e.g., radio frequency identification (RFID), Zigbee communication protocol, WiFi, infrared, wireless Universal Serial Bus (USB), Ultra Wide Band (UWB), Bluetooth^{®} communication protocol, and cellular communications, such as Code Division Multiple Access (CDMA) or the Global System for Mobile Communications (GSM).

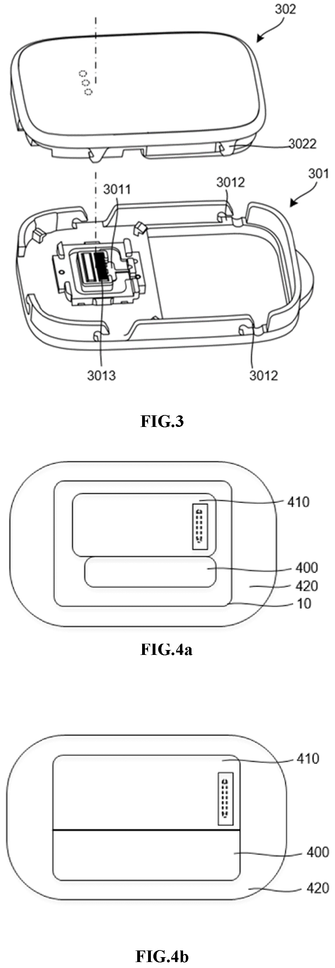

FIG.2 is a schematic diagram of the integrated continuous glucose monitoring device according to an embodiment of the present invention. FIG.3 is a schematic diagram of the split continuous glucose monitoring device according to an embodiment of the present invention.

The CGM comprising a sensor and a transmitter, mounted on the patient by means of an auxiliary mount, pierced subcutaneously. The sensor is used to collect analyte data in the human body and transmit the collected analyte content information. The transmitter is connected to the sensor, and it is used to receive the information on the blood glucose data, which is transmitted by the sensor that has been implanted under the skin, and convert the information into a wireless signal for outputting. Each CGM has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the CGM, or may also be set differently depending on the type of the CGM.

FIG.2 is a schematic diagram of the integrated continuous glucose monitoring device, i.e., the sensor and the transmitter have been integrated together prior to use, and is a single-use product, which is discarded after use, as shown in FIG. 2. The integrated continuous glucose monitoring device includes a sensor 201, a housing 202, and a transmitter (not shown in the figure) set inside the housing 202, the sensor 301 is used to monitor the patient's body fluid blood glucose data information, and transmit the said blood glucose data information to the transmitter through an internal circuit, which in turn sends it to a receiver. The identifier may be provided on the outer housing of the CGM or on the outer packaging or within the CGM.

FIG.3 is a schematic diagram of the split continuous glucose monitoring device, i.e., the sensor and the transmitter are two different parts prior to use, packaged separately, and only integrated together when in use, as shown in FIG. 3. The split continuous glucose monitoring device includes a base 301 and a transmitter 302, the base is provided with a sensor 3011, the transmitter 302 has a separate housing, the housings of the base 301 and the transmitter 302 are respectively provided with snap-in structures 3012 and 3022, the base 301 and the transmitter 302 are snapped into a whole through the snap-in structures when in use, the sensor 3011 is electrically connected to the transmitter 302 through the electrical connection 3013, the sensor 301 is used to monitor the patient's blood glucose data information, the blood glucose data information is transmitted to the transmitter 302 through the electrical connection 3013, and then sent to the receiver by the transmitter 302.

In one embodiment of the present invention, both the sensor and the transmitter of the split continuous glucose monitoring device are single-use products, which are discarded after use, and therefore the identifier may be provided on the housing or the outer packaging of the sensor or the transmitter. In yet another embodiment of the present invention, only the sensor of the split continuous glucose monitoring device is a single-use product and the transmitter is a reusable product, therefore, preferably, in this embodiment, the identifier is provided on the housing or the outer packaging of the transmitter, which reduces the frequency of tying the patient's information to the identifier and improves the patient experience. This will be described in more detail below.

When the identifier is set on the housing or outer packaging of the CGM or transmitter, it is set in a form that includes, but is not limited to, a QR code, a bar code, or an NFC tag.

FIG.4a is a schematic diagram of the integrated insulin pump according to an embodiment of the present invention. FIG.4b is a schematic diagram of the split insulin pump according to an embodiment of the present invention.

In this embodiment of the invention, the insulin pump is a patch-type insulin pump, i.e., an insulin pump that does not comprise a long catheter, includes an infusion mechanism and a control mechanism, and being integrally affixed to the surface of the user's skin by an adhesive patch, and the medication is infused directly from the reservoir to the subcutaneous area along the infusion needle.

Each insulin pump has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the insulin pump, or may also be set differently depending on the type of the insulin pump.

FIG.4a is a schematic diagram of the integrated insulin pump, i.e., the infusion mechanism 410 and the control mechanism 400 of the insulin pump are provided inside the same housing 10, and both are connected by wires and are affixed to a location on the user's skin by means of an adhesive patch 420, which is disposed of in its entirety after a single use; an identifier may be provided on the outer housing of the insulin pump or on the outer packaging or inside the insulin pump.

FIG.4b is a schematic diagram of the split insulin pump, i.e., the infusion mechanism 410 and the control mechanism 400 of the insulin pump are provided in two different housings, both of which are connected by waterproof plugs or directly snap together and electrically connected as a whole, an identifier may be provided on the outer housing of the infusion mechanism and/or the control mechanism or on the outer packaging or inside the insulin pump.

In one embodiment of the present invention, both the infusion mechanism and the control mechanism of the split insulin pump are single-use products, which are discarded after use, and therefore the identifiers may be provided on the housing or on the outer packaging of the infusion mechanism and/or the control mechanism. In yet another embodiment of the present invention, only the infusion mechanism of the split insulin pump is a single-use product, while the control mechanism is a reusable product, and therefore, preferably, in this embodiment, the identifier is set on the housing of the control mechanism or on the outer packaging, which can reduce the frequency of binding of patient information and identifiers, and improve the patient experience. This is described in more detail below.

When the identifier is set on the housing of the control mechanism or on the outer packaging, it is set in a form that includes, but is not limited to, a QR code, a bar code, or an NFC tag.

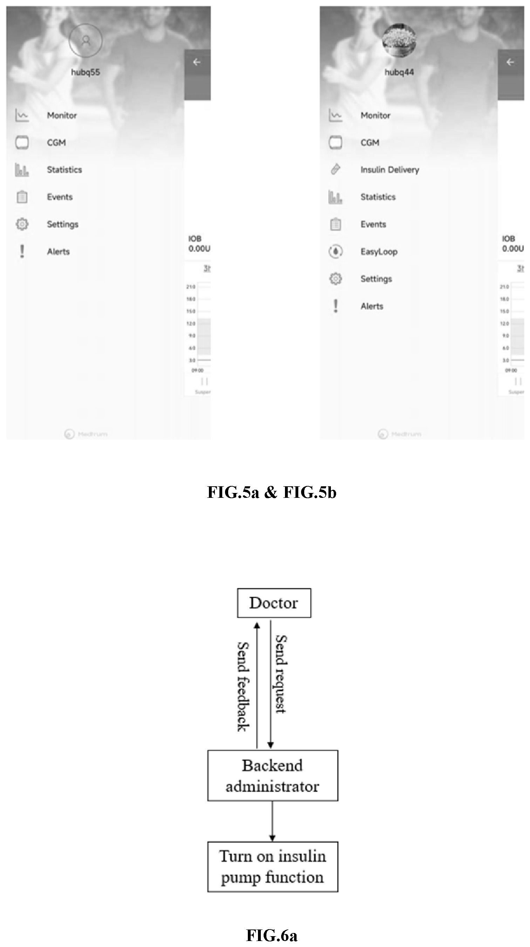

As mentioned earlier, considering the severity of illness in patients and the individual patient's physical health status, some patients may only need a CGM for continuous glucose monitoring, while others may need not only a CGM for continuous glucose monitoring, but also an insulin pump for drug infusion. When a doctor determines that a patient only needs to use a CGM for continuous glucose monitoring, and as the CGM involves only the monitoring of the patient's user's blood glucose, and the user's own use of the CGM does not pose a risk to the user's life and safety, the users may purchase the CGM by themselves. Before the CGM is installed on the surface of the user's skin, the user can search for and download the dedicated APP for controlling the CGM from the smartphone's app store, create a new account on the dedicated APP, and pair the patient's personal information with the information of the CGM to be worn, thus realizing the pairing and controlling of the smartphone and the CGM. The CGM and the insulin pump in the embodiment of the present invention are developed and produced by the same manufacturer, and thus can be controlled by the same dedicated APP in the smartphone, and since the insulin pump is not required to be used by all patients, only CGM-related content is involved in the default main screen of the dedicated APP, as shown in FIG. 5a. On the one hand, it can simplify the interface of the APP and provide the user with a good visual experience, and on the other hand, prevent the user from mis-operation on the insulin pump, which may affect the normal use of the CGM.

In one embodiment of the present invention, when a doctor determines that a patient needs to use an insulin pump for drug infusion, as shown in FIG. 6a, the doctor sends a request to the backend administrator to add the patient's account to a whitelist to allow the patient to use the insulin pump function, and the backend administrator receives the request to add a whitelist sent by the doctor and adds the patient's account to a list of whitelists, and at the same time sends the doctor a feedback that the addition of the whitelist has been completed, and furthermore the backend administrator turns on directly the insulin pump function on the app interface that the patient is using. At this time, the APP interface changes from FIG. 5a to FIG. 5b, and the interface of FIG. 5b adds two function keys related to insulin infusion, "Insulin Delivery" and "Easyloop", compared with the interface of FIG. 5a. In another embodiment of the present invention, the backend administrator does not directly turn on the insulin pump function of the APP interface used by the patient, but sends a security code to the patient's account, so that the patient can turn on the insulin pump function on the APP interface through the security code when needed or convenient.

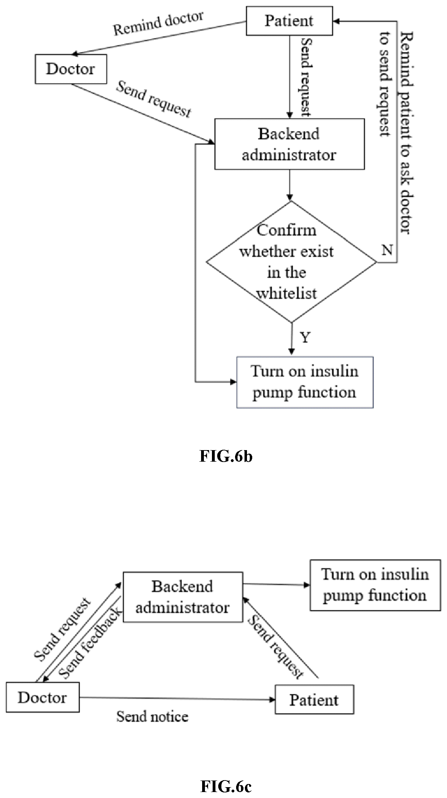

In another embodiment of the present invention, when the doctor determines that the patient needs to use the insulin pump for drug infusion, as shown in FIG. 6b, the patient can send an request to turn on the insulin pump function directly to the backend administrator, and the backend administrator receives the request to turn on the insulin pump function sent by the patient and verifies that whether the patient's account is in the whitelist or not, and if the patient's account is in the whitelist, the backend administrator turns on the insulin pump function in the APP interface that the patient is using and the APP interface changes from FIG. 5a to FIG. 5b; If the patient's account is not in the whitelist, a feedback message is sent to the patient's account to remind the patient to ask the doctor to send an add whitelist request to the backend administrator. After the doctor sends the whitelist request to the background administrator, the background administrator can directly turn on the insulin pump function of the APP interface used by the patient. If no message is received from the background administrator within a certain period of time, such as 1min, 2min, 5min, then another request for turning on the insulin pump function to the backend administrator can be send again, or ask the doctor to send the request for adding whitelist to the backend administrator. In another embodiment of the present invention, the backend administrator does not directly turn on the insulin pump function on the APP interface used by the patient, but instead sends a security code to the patient's account, which allows the patient to turn on the insulin pump function of the APP interface through the security code when needed or convenient.

In yet another embodiment of the present invention, when the doctor determines that the patient needs to use the insulin pump for drug infusion, as shown in FIG. 6c, the doctor sends a request to the backend administrator to add the patient account to the whitelist to allow the patient to use the insulin pump function, and the backend administrator receives the request sent by the doctor to add a whitelist and adds the patient account to the whitelist, and at the same time sends the doctor a feedback that the whitelist addition has been completed, and further the doctor notifies the patient that the patient can apply to use the insulin pump function. After receiving the notification from the doctor, the patient sends a request for turning on the insulin pump function to the backend administrator, and after the backend administrator receives the request for turning on the insulin pump function sent by the patient, the backend administrator directly turns on the insulin pump function on the APP interface used by the patient. In another embodiment of the present invention, after the backend administrator receives the request to turn on the insulin pump function sent by the patient, the backend administrator may also first verify whether the patient account exists in the whitelist or not, and if it is determined that the patient account is in the whitelist, the backend administrator turns on the insulin pump function on the APP interface used by the patient. In another embodiment of the present invention, the backend administrator does not directly turn on the insulin pump function on the APP interface used by the patient, but instead sends a security code to the patient's account, through which the patient can turn on the insulin pump function on the APP interface when needed or convenient.

When the doctor determines that the patient no longer needs to turn on the insulin pump function, the patient can turn off the insulin pump function on the APP by himself/herself, and the APP automatically sends a message to the backend administrator, who deletes the patient's account from the whitelist; the doctor and/or the patient can also send an request to the backend administrator to request to turn off the insulin pump function, and the backend administrator will turn off the insulin pump function on the APP and at the same time delete that patient account from the whitelist. When this patient needs to turn on the insulin pump function again, the patient account needs to be re-added to the whitelist by referring to one of methods as shown in FIG. 6a- FIG.6c.

It should be noted that it is necessary to ensure that the insulin pump is properly mounted on the skin before turning on the insulin pump function of the APP, and to pair the patient's personal information with the information of the insulin pump, so as to realize the pairing and controlling of the smartphone and the insulin pump. The personal information of the patient includes name, age, gender, cell phone number, etc., and the information of the CGM and/or insulin pump worn includes the identifier information of the CGM and/or insulin pump. At the same time, the smartphone uploads the personal information of the patient and the identifier information of the CGM and/or the insulin pump to a remote server, which can store the information uploaded by the smartphone and verify whether the identifier information of the CGM and/or the insulin pump is valid, and if a certain identifier information already exists in the remote server, the remote server sends an alert to the smartphone to remind the user that the CGM or insulin pump has been used and needs to be replaced. When the CGM and/or the insulin pump is mounted on the skin of the patient and successfully activated, the CGM and/or the insulin pump starts to work, the transmitter of the CGM sends the monitored blood glucose information to the smartphone and further uploads it to the remote server, and the control mechanism of the insulin pump receives the insulin infusion information and controls the infusion mechanism to infuse the insulin, and at the same time sends the infusion status to the smartphone and further uploads it to the remote server.

It should be noted that the CGM and insulin pump in the embodiment of the present invention are developed and produced by the same manufacturer, and thus can be controlled by the same dedicated APP in the smartphone. Assuming that CGM or insulin pumps produced by other manufacturers also can be controlled by a dedicated APP in the smartphone, and the inconvenience to the user due to the use of different APPs for controlling the CGM and the insulin pump respectively can be avoided, and the user experience can be improved.

When the CGM and/or insulin pump worn by the patient needs to be replaced for reasons such as reaching the end of its life cycle or failing, the unique identifier information of the new CGM and/or insulin pump also needs to be updated by pairing with the patient's personal information via the smartphone and further uploaded to a remote server. The patient's personal information is entered manually, and the identifier information of the CGM and/or insulin pump may also be entered manually or by scanning a QR code, a barcode, or NFC tags on the housing or on the outer packaging of the CGM and/or the insulin pump.

When the CGM is split and the transmitter is reusable, the identifier of the CGM is set on the housing or packaging of the transmitter, so that the patient only needs to replace the sensor when replacing the CGM without replacing the transmitter, and the identifier of the CGM remains unchanged, and thus there is no need for updating the pairing of the identifier of the CGM with the personal information of the patient via a smartphone, and no need for uploading it to a remote server, and thus the number of operational steps can be reduced to improve the patient experience.

When the insulin pump is split and the control mechanism is reusable, the identifier of the insulin pump is set on the housing or packaging of the control mechanism, and the patient only needs to change the infusion mechanism when replacing the insulin pump without changing the control mechanism, and the identifier of the insulin pump remains unchanged, and thus there is no need for updating the pairing update of the identifier of the insulin pump with the personal information of the patient via the smartphone or in uploading it to a remote server, and thus the operational steps can be reduced and the patient experience can be improved.

The smartphone and the CGM and/or insulin pump, and the remote server communicate with each other via wireless communication, which may be via, for example, but not limited to, radio frequency (RF) communication (e.g., radio frequency identification (RFID), Zigbee communication protocols, WiFi, infrared, wireless Universal Serial Bus (USB), Ultra Wide Band (UWB), Bluetooth^{®} communication protocols, and cellular communication, such as Code Division Multiple Access (CDMA) or Global System for Mobile Communications (GSM). communications, such as Code Division Multiple Access (CDMA) or Global System for Mobile Communications (GSM). Preferably, the smartphone and the remote server communicate with each other via WiFi and/or cellular communication, and the smartphone and the CGM and/or insulin pump communicate with each other via Bluetooth^{®} communication protocol.

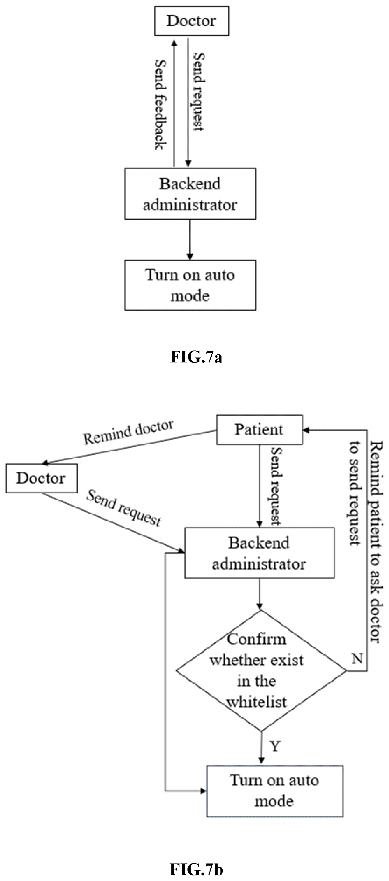

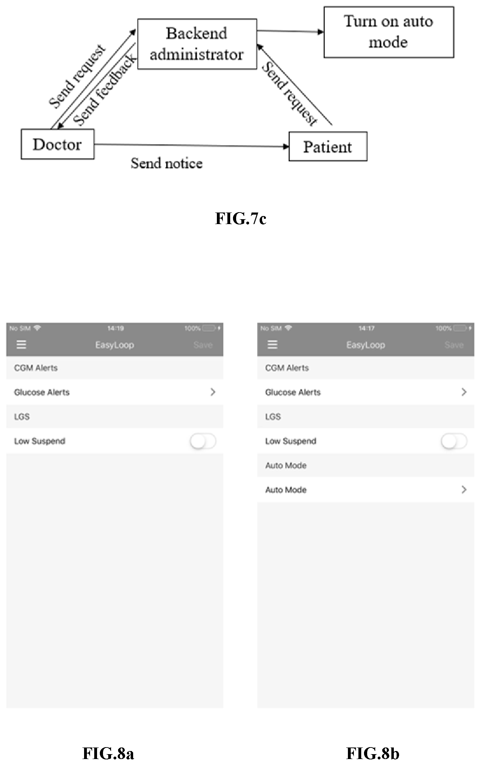

When the doctor determines that the patient can turn on the automatic mode, i.e. after the APP reads the current blood glucose value monitored by the CGM and the insulin information infused by the insulin pump, it can predict the future trend of blood glucose and control the infusion of the insulin pump based on the predicted trend of blood glucose, including increasing, decreasing, or stopping the infusion of insulin, in order to affect the blood glucose values, and form an automated closed-loop control cycle. As shown in FIG. 7a, the doctor sends a request to the backend administrator to add the patient account to the whitelist to allow the patient to use the auto mode function, and the backend administrator receives the add whitelist request sent by the doctor and adds the patient account to the whitelist, and at the same time sends the feedback to the doctor that the whitelist has been added, and furthermore directly turns on the auto mode function on the APP interface used by the patient. At this time, the APP interface changes from FIG. 8a to FIG. 8b, and the interface of FIG. 8b has added "Auto Mode", i.e., a function key related to the auto mode, compared with the interface of FIG. 8a. In another embodiment of the present invention, the backend administrator does not directly turn on the Auto Mode function on the APP interface used by the patient, but sends a security code to the patient's account, so that the patient can turn on the Auto Mode function on the APP interface through the security code when needed or convenient.

In another embodiment of the present invention, when the doctor determines that the patient needs to use the insulin pump for drug infusion, as shown in FIG. 7b, the patient can send a request to turn on the auto mode function directly to the backend administrator, and the backend administrator, upon receipt of the request sent by the patient to turn on the auto mode function, verifies whether the patient's account is in the whitelist or not, and if the patient's account is in the whitelist, the backend administrator turns on the auto mode function on the APP interface that is used by the patient, and the APP interface changes from FIG. 8a to FIG. 8b. If the patient's account is not in the whitelist, a feedback message is sent to the patient's account to remind the patient to ask the doctor to send an add whitelist request to the background administrator. After the doctor sends the whitelist request to the backend administrator, the backend administrator can directly turn on the auto mode function on the APP interface used by the patient. If no message is received from the backend administrator within a certain period of time, such as 1min, 2min, 5min, another request for turning on the insulin pump function to the backend administrator can be sent again, or ask the doctor to send the request for adding whitelist to the backend administrator. In another embodiment of the present invention, the backend administrator does not directly turn on the auto mode function on the APP interface used by the patient, but instead sends a security code to the patient's account, through which the patient can turn on the auto mode function on the APP interface when needed or convenient.

In yet another embodiment of the present invention, when the doctor determines that the patient needs to use the insulin pump for drug infusion, as shown in FIG. 7c, the doctor sends a request to the backend administrator to add the patient account to the whitelist to allow the patient to use the auto mode function, and the backend administrator receives the request to add a whitelist sent by the doctor and adds the patient account to the whitelist, and at the same time sends a feedback to the doctor that the whitelist has been added, and furthermore the doctor notifies the patient that he/she can request to use the auto mode function. After receiving the notification from the doctor, the patient sends a request for turning on the auto mode function to the backend administrator, and after the backend administrator receives the request for turning on the auto mode function sent by the patient, the backend administrator directly turns on the auto mode function on the APP interface used by the patient. In another embodiment of the present invention, after the backend administrator receives the request for turning on the auto mode function sent by the patient, the backend administrator may also first verify whether the patient account exists in the whitelist or not, and if it is determined that the patient account is in the whitelist, the backend administrator turns on the auto mode function on the APP interface used by the patient. In another embodiment of the present invention, the backend administrator does not directly turn on the auto mode function on the APP interface used by the patient, but instead sends a security code to the patient's account, through which the patient can turn on the auto mode function on the APP interface when needed or convenient.

When the doctor determines that the patient no longer needs to turn on the auto mode function, the patient can turn off the auto mode function on the APP by themselves, and the APP automatically sends a message to the backend administrator, who deletes the patient account from the whitelist. Or the doctor and/or the patient can also send a request to the backend administrator to turn off the auto mode function, and the backend administrator turns off the auto mode function on the APP, and at the same time deletes the patient account from the whitelist. When this patient needs to turn on the auto mode function again, the patient account needs to be re-added to the whitelist by referring to one of the methods as shown in FIG. 7a-FIG. 7c.

In embodiments of the present invention, the security code sent by the backend administrator when applying for insulin pump function and auto mode function can be any one or combination of a series of numeric characters, alphabetic characters, and other symbols, as well as a series of taps, a series of inputs, complex or simple gestures (e.g., swipes or other movements on a touch screen, drawing images), and the like. In some cases, the security code may also include a quiz or set of questions. Each security code sent by the backend administrator is randomized.

Given the considerable variation in dietary habits across different regions and age groups, a standardized control system may lead to suboptimal glycemic management for certain individuals, when the patient's auto mode function is turned on, the patient is asked to enter a pass code to access the different auto mode interfaces.

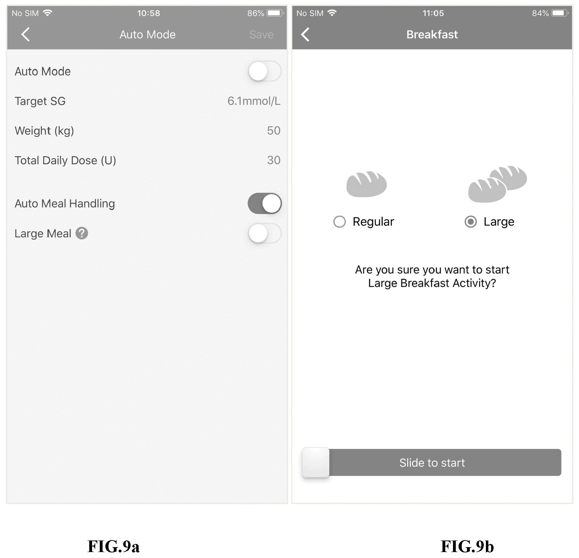

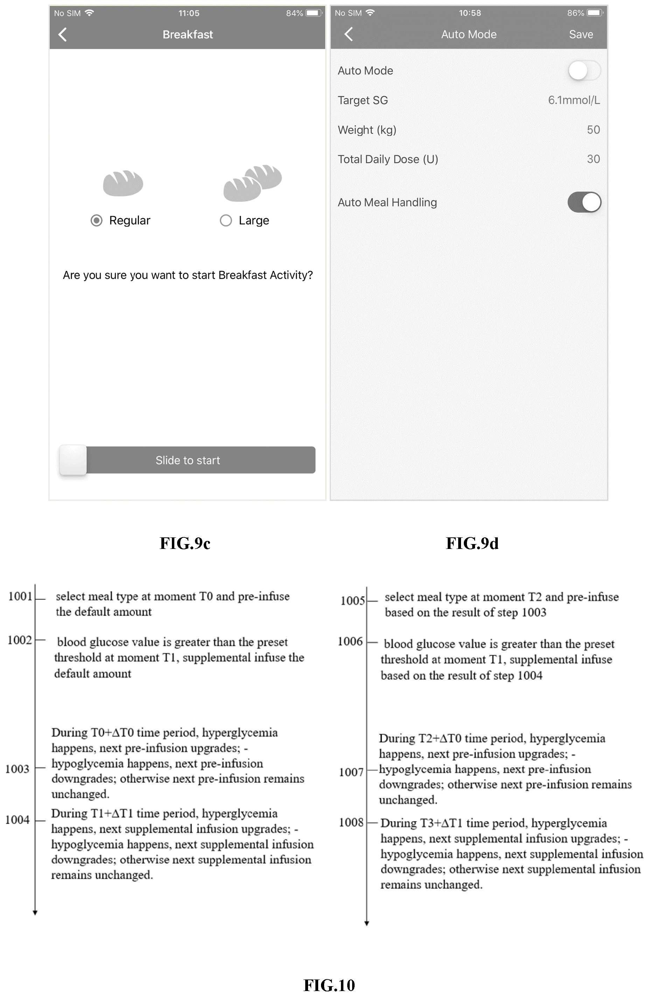

For people with high carbohydrate consumption, entering the pass code to access the high carbohydrate mode, and the interface of the auto mode is shown in FIG. 9a, a large meal option displays on the interface of the high carbohydrate mode, the patient can choose whether to turn on the function of the Large Meal option, after the patient turns on the function of the Large Meal option, two options of "Regular" and "Large" will be displayed on the infusion page, as shown in Fig. 9b and FIG. 9c. When the patient selects "regular ", the system will inject at least the amount of insulin corresponding to the amount of regular carbohydrates; when the patient selects "Large ", the system will inject at least the amount of insulin corresponding to a larger amount of carbohydrates. For people with normal carbohydrate consumption, after entering the pass code, the system enters the normal carbohydrate mode, the interface of the auto mode is shown in FIG. 9d, the interface of the normal carbohydrate mode does not have the option of "Large Meal", and the default is the " Regular " mode, and the system will infuse at least the amount of insulin corresponding to the amount of regular carbohydrates.

In embodiments of the present invention, the pass code may be a small set of questions, such as "Are you a carb enthusiast?", "Is your age in the A-B range?", "What is your gender?", "Where do you live?", "What are your fitness preferences?", "Are there any special medical conditions?", "Have you uses the non-auto mode before turning on the auto mode?" etc. Based on the patient's answers, the system automatically determines whether the patient is a high carbohydrate consumer. In other embodiments of the present invention, the pass code is communicated to the patient by a doctor after diagnosing, or the doctor sends information about whether the patient is a high carbohydrate consumer while sending a whitelist request for the auto mode to the backend administrator, and the backend administrator automatically assigns the patient the corresponding pass code, which may be anyone or combination of a series of numeric characters, alphabetic characters, and other symbols, as well as a series of taps, a series of inputs, complex or simple gestures (e.g., swipes or other movements on a touch screen, drawing images), and the like, and the backend administrator may send the pass code to the patient when turning on the auto mode, or send the pass code to the patient at the same time as sending the security code, or send the pass code to the patient after confirming that the patient has turned on the auto mode via the security code. In the embodiment of the present invention, both the security code and the pass code are randomly generated, and their generation rules may or may not be the same, and preferably, the generation rules of the security code and the access code are not the same, so as to avoid confusion of the patient and thus causing disturbance to the patient.

In embodiments of the present invention, the system or the doctor's judgment of whether the patient is a high carbohydrate consumer may be the result of a comprehensive judgment based on a plurality of conditions, such as the patient's gender, age, dietary habits, exercise habits, health status, insulin sensitivity, carbohydrate sensitivity, and results of the use of the non-auto mode, and when the patient has a record of the use of the non-auto mode, the result of the non-auto mode is used as the primary basis for the judgment.

When the patient selects a meal, including "Regular" and "Large" meals, the auto mode uses the drug infusion strategy of pre-infusion and supplemental infusion, as shown in FIG 10. In pre-infusion and supplemental infusion strategy, the estimated meal size corresponding to the pre-infusion amount and supplemental infusion amount were categorized into different levels for both "Regular" and "Large" meals, as shown in following Table 1:

| Meal option | Estimated meal size for pre-infusion | Estimated meal size for supplemental infusion |
|---|---|---|
| Regular | Small (default) | Small (default) |
| | Middle | Middle |
| | Big | Big |
| Large | Small (default) | Small (default) |
| | Middle | Middle |
| | Big | Big |

Wherein, the insulin amount for the pre-infusion is at least related to the actual blood glucose value or its rate of change at the time of pre-infusion, and the estimated meal size for pre-infusion. In other embodiments of the present invention, the insulin amount for the pre-infusion may also be related to the IOB in the body. Similarly, the insulin amount for the supplemental infusion is at least related to the actual blood glucose value or its rate of change at the time of supplemental infusion, and estimated meal size for the supplemental meal. In other embodiments, the insulin amount for supplemental infusion may also be related to the IOB in the body. In embodiments of the present invention, the meal size is the amount of carbohydrate contained in the meal.

The estimated meal sizes (Small, Middle, Big) for the pre-infusion and the supplemental infusion are independent parameters. For the same level, the estimated meal size for the pre-infusion is greater than the estimated meal size for the supplemental infusion, but there is not necessarily a fixed correspondence between the two, and the system can be set according to the actual needs. For the Large meal, the minimum value of the estimated meal size for the pre-infusion is not less than the maximum value of the Regular meal, and the minimum value of the estimated meal size for the supplemental infusion is not less than the maximum value of the Regular meal. In embodiments of the present invention, in pre-infusion, the range of estimated meal sizes for Regular meals that may be 40-80g, 30-60g, etc., and for Large meals that may be 80-120g, 60-100g, etc., and then according to certain rules to divide for Big, Middle, Small, or more levels, respectively. In supplemental infusion, the range of estimated meal sizes for Regular meals that may be 20-40g, 10-30g, etc., and for Large meals that may be 40-60g, 30-50g, etc., and then according to certain rules to divide for Big, Middle, Small, or more levels, respectively. In other embodiments of the present invention, the range of estimated meal sizes for Regular and Large meals at the time of pre-infusion or at the time of supplemental infusion may also be any other reasonable range, which is then according to certain rules to divide for Big, Middle, Small, or more levels, respectively.

FIG. 10 is a schematic diagram of the process of an infusion strategy for pre-infusion and supplemental infusion according to embodiments of the present invention.

Step 1001, the patient selects a meal type at the moment of T0, and a default insulin amount is pre-infused, and the pre-infused default insulin amount may be corresponding to any one of the estimated meal sizes for pre-infusion, Big, Middle, or Small, and preferably, the pre-infused default insulin amount is corresponding to the Small size, and selecting a small amount of the initial pre-infused insulin amount can prevent too much insulin from being infused, and reduces the risk of causing hypoglycemia.

Step 1002, at the moment T1, comparing the current blood glucose value monitored by the CGM with a preset blood glucose threshold, e.g., 140, 150, 160, 170, 180, 190, 200 mg/mL, etc., if the current blood glucose value is greater than the preset blood glucose threshold, the default supplemental insulin amount is infused, otherwise, the supplemental infusion amount is not infused, and the default supplemental insulin amount may be corresponding to any one of the estimated meal sizes for supplemental infusion, Small, Middle, or Big, and preferably, the default supplemental insulin amount is corresponding to the small size, and selecting a small amount of the initial supplemental infused insulin amount can prevent too much insulin from being infused, and reduces the risk of causing hypoglycemia. The T1 moment is a moment later than the T0 moment, such as 1h, 1.5h, 2h, 2.5h, 3h, etc. In other embodiments of the present invention, the rate of change of blood glucose at the T1 moment may also be combined to determine whether to administer a supplemental infusion.

Step 1003, within ΔT0 time period after the T0 moment, e.g., within 3h, 4h, 5h, etc., if hyperglycemia happened, the next pre-infusion will be upgraded, i.e., the amount of insulin corresponding to a bigger estimated meal size will be infused, at the same time, taking into account the actual glucose value or rate of change of glucose, or IOB at the moment of the next pre-infusion. If hypoglycemic happened, the next pre-infusion will be downgraded, i.e., the amount of insulin corresponding to a smaller estimated meal size will be infused, at the same time, taking into account the actual glucose value or rate of change of glucose, or IOB at the moment of the next pre-infusion. If no hyperglycemia or hypoglycemic happened, the level of the next pre-infusion will be maintained, i.e., the amount of insulin corresponding to the same estimated meal size will be is infused, at the same time, taking into account the actual glucose value or rate of change of glucose, or IOB at the moment of the next pre-infusion.

It should be noted that in embodiments of the present invention, an adjustment in the infusion level implies an adjustment in the level of the estimated meal size at the time of the infusion, at the same time, taking into account the actual blood glucose value or rate of change of blood glucose, or IOB at the moment of the infusion, and therefore, in embodiments of the present invention, an adjustment in the level of the estimated meal size implies an adjustment in the level of the infusion, i.e., the level of the infusion and the level of the estimated meal size may be understood to be the same.

Step 1004, within ΔT1 time period after the T1 moment, e.g., within 3h, 4h, 5h, etc., if hyperglycemia happened, the next supplemental infusion will be upgraded, i.e., the amount of insulin corresponding to a bigger estimated meal size will be infused, at the same time, taking into account the actual glucose value or rate of change of glucose, or IOB at the moment of the next s supplemental infusion. If hypoglycemic happened, the next supplemental infusion will be downgraded, i.e., the amount of insulin corresponding to a smaller estimated meal size will be infused, at the same time, taking into account the actual glucose value or rate of change of glucose, or IOB at the moment of the next supplemental infusion. If no hyperglycemia or hypoglycemic happened, the level of the next supplemental infusion will be maintained, i.e., the amount of insulin corresponding to the same estimated meal size will be infused, at the same time, taking into account the actual glucose value or rate of change of glucose, or IOB at the moment of the next supplemental infusion.

Step 1005, the patient selects the meal type at the T2 moment, and pre-infuses the amount of insulin corresponding to a bigger, smaller, or unchanged estimated meal size based on the results of step 1003, and taking into account the actual blood glucose value or the rate of change of blood glucose or the IOB at the moment of T2.

Step 1006, at the moment T3, comparing the current blood glucose value monitored by the CGM with a preset blood glucose threshold, e.g., 140, 150, 160, 170, 180, 190, 200 mg/mL, etc., if the current blood glucose value is greater than the preset blood glucose threshold, supplemental infuses the amount of insulin corresponding to a bigger, smaller, or unchanged estimated meal size based on the results of step 1004, and taking into account the actual blood glucose value or the rate of change of blood glucose or the IOB at the moment of T3. if the current blood glucose value is not greater than the preset blood glucose threshold, no supplemental infuse is performed. In other embodiments of the present invention, the rate of change of blood glucose at the T3 moment may also be combined to determine whether to administer a supplemental infusion.

Step 1007, within ΔT2 time period after the T2 moment, e.g., within 3h, 4h, 5h, etc., if hyperglycemia happened, the next pre-infusion will be upgraded, i.e., the amount of insulin corresponding to a bigger estimated meal size will be infused, at the same time, taking into account the actual glucose value or rate of change of glucose, or IOB at the moment of the next pre-infusion. If hypoglycemic happened, the next pre-infusion will be downgraded, i.e., the amount of insulin corresponding to a smaller estimated meal size will be infused, at the same time, taking into account the actual glucose value or rate of change of glucose, or IOB at the moment of the next pre-infusion. If no hyperglycemia or hypoglycemic happened, the level of the next pre-infusion will be maintained, i.e., the amount of insulin corresponding to the same estimated meal size will be infused, at the same time, taking into account the actual glucose value or rate of change of glucose, or IOB at the moment of the next pre-infusion.

Step 1008, within ΔT1 time period after the T3 moment, e.g., within 3h, 4h, 5h, etc., if hyperglycemia happened, the next supplemental infusion will be upgraded, i.e., the amount of insulin corresponding to a bigger estimated meal size will be infused, at the same time, taking into account the actual glucose value or rate of change of glucose, or IOB at the moment of the next s supplemental infusion. If hypoglycemic happened, the next supplemental infusion will be downgraded, i.e., the amount of insulin corresponding to a smaller estimated meal size will be infused, at the same time, taking into account the actual glucose value or rate of change of glucose, or IOB at the moment of the next supplemental infusion. If no hyperglycemia or hypoglycemic happened, the level of the next supplemental infusion will be maintained, i.e., the amount of insulin corresponding to the same estimated meal size will be infused, at the same time, taking into account the actual glucose value or rate of change of glucose, or IOB at the moment of the next supplemental infusion.

Repeat steps 1005-1008 when pre-infusion and supplemental infusion is required at the next moment.

Generally, according to the patient's dietary habits, and in order to maintain the stability of the patient's blood glucose level, the patient chooses the same meal type at the moment of T2 and T0, i.e., if the patient chooses Regular at the moment of T0, he/she also chooses Regular at the moment of T2. If Large is selected at the moment of T0, Large meal is also selected at T2, so that the next pre-infusion insulin selection can be dependent on the results of the previous pre-infusion insulin selection, and the next supplemental insulin infusion selection can also be dependent on the results of the previous supplemental insulin infusion selection, except that, if the patient's choice of the next meal is inconsistent with that of the previous one, e.g., when the next meal selection is not the same as the previous one for the first time, the patient is reverted back to the initial default level for the next pre-infusion and supplemental infusion in order to prevent inaccuracy of the amount of insulin infusion due to change in the mode of the meal. When the meal choice is different again, i.e., when the next two choices revert back to being the same as the previous choice, the levels at the next two pre-infusions and supplemental infusion are the same as the pre-infusion and supplemental infusion levels of the previous one, based on the same infusion levels as the previous one, which results in a more accurate insulin infusion at the current meal and better control of the patient's blood glucose level. For example, assuming that the patient's previous five choices were all Regular, in which the pre-infusion and supplemental infusion levels were both Middle, and that when the choice for the 6th choice was Large (the choices are different for the first time), the pre-infusion and supplemental infusion levels at the 6th choice were the default levels; and that when the patient's choice for the 7th choice reverted back to Regular (the choices are different again), the pre-infusion and supplemental infusion levels at the 7th choice were the same as those that had been used at the time of the pre-infusion and supplemental infusion at the 5th choice, i.e., that is Regular, and the level is Middle.

Here, it should be noted that if the current pre-infusion amount corresponding to the estimated meal size is already the biggest estimated meal size in the selected meal type, and if hyperglycemia still occurs during the ΔT0 time period after the T0 or T2 moments, the next pre-infusion amount will not be upgraded, and the amount of insulin corresponding to the biggest (Big in here) estimated meal size will still be pre-infused, taking into account the actual glycemic value or the rate of change in glycemia or the IOB at the moment of the next pre-infusion. Similarly, if the current pre-infusion amount corresponding to the estimated meal size is already the smallest estimated meal size in the selected meal type, and if hypoglycemia still occurs during the ΔT0 time period after the T0 or T2 moments, the next pre-infusion amount will not be downgraded, and the amount of insulin corresponding to the smallest (Small in here) estimated meal size will still be pre-infused, taking into account the actual glycemic value or the rate of change in glycemia or the IOB at the moment of the next pre-infusion. That is, it limits the safe infusion boundaries for insulin infusion and effectively prevents hyperglycemia and hypoglycemia.

If the current supplemental infusion amount corresponding to the estimated meal size is already the biggest estimated meal size in the selected meal type, and if hyperglycemia still occurs during the ΔT1 time period after the T1 or T3 moments, the next supplemental infusion amount will not be upgraded, and the amount of insulin corresponding to the biggest (Big in here) estimated meal size will still be supplemental infused, taking into account the actual glycemic value or the rate of change in glycemia or the IOB at the moment of the next supplemental infusion. Similarly, if the current supplemental infusion amount corresponding to the estimated meal size is already the smallest estimated meal size in the selected meal type, and if hypoglycemia still occurs during the ΔT1 time period after the T1 or T3 moments, the next supplemental infusion amount will not be downgraded, and the amount of insulin corresponding to the smallest (Small in here) estimated meal size will still be supplemental infused, taking into account the actual glycemic value or the rate of change in glycemia or the IOB at the moment of the next supplemental infusion. That is, it limits the safe infusion boundaries for insulin infusion and effectively prevents hyperglycemia and hypoglycemia.

In other embodiments of the present invention, the corresponding estimated meal size at the time of pre-infusion and at the time of supplemental infusion are not necessarily both levels, i.e., the estimated meal size at the time of pre-infusion may be only the default level, while at the time of supplemental infusion, the estimated meal size is of three different levels of Big, Middle and Small, respectively, and therefore at the pre-infusion stage, only the amount of insulin corresponding to the default meal size is infused; or the estimated meal size at the time of pre-infusion has three different levels of Big, Middle and Small, while at the time of the supplemental infusion, the estimated meal size is only the default level, and thus at each time of supplemental infusion only the amount of insulin corresponding to the default meal size is infused.

Similarly, the level settings for the estimated meal size corresponding to pre-infusion and supplemental infusion are not necessarily the same in Large and Regular, i.e., for each meal type, the estimated meal size corresponding to pre-infusion and supplemental infusion can be selected to be levels or ungraded (the default size), which can be set according to the patient's actual needs.

In other embodiments of the present invention, the high carbohydrate mode and the normal carbohydrate mode also include a small carbohydrate mode, such as the small carbohydrate mode can be selected when the patient eats a snack, and when the patient selects the small carbohydrate mode, the system performs the insulin infusion based on the default estimated meal size because of the small range of the carbohydrate content in the small carbohydrate, and at the same time, taking into account the actual glycemic value or rate of change of the blood glucose, or IOB of the patient when he/she eats the snack, and the default estimated meal size in the small carbohydrate mode is less than the lowest level of the estimated meal size in the normal mode.

In summary, the present invention discloses a closed-loop artificial pancreas system, comprising: a detection mechanism, an infusion mechanism, and a program mechanism, at least based on the blood glucose value detected by the detection mechanism, the program mechanism generates an insulin infusion instruction and controls the infusion mechanism to perform the infusion, the system utilizes an infusion strategy of pre-infusion and supplemental infusion, and an amount of insulin for pre-infusion is related to an estimated meal size at a time of pre-infusion, an amount of insulin for supplemental infusion is related to an estimated meal size at a time of supplemental infusion. There is no need to explicitly determine the size of the meal, and the system determines the amount of insulin to be infused based on the size of the predicted meal, on the one hand, relieving the pressure to accurately determine the size of the meal, and at the same time, taking into account the dynamic impact on blood glucose levels after the meal and the infusion of a bolus, the system adopts the infusion strategy of pre-infusion and supplemental infusion, so as to keep the patient's glucose concentration at a desirable level.

While the invention has been described in detail with reference to the specific embodiments of the present invention, it should be understood that it will be appreciated by those skilled in the art that the above embodiments may be modified without departing from the scope and spirit of the invention. The scope of the invention is defined by the appended claims.

## Claims

1. A closed-loop artificial pancreas system, comprising:
a detection mechanism, configured to continuously detect a blood glucose value of a patient;
an infusion mechanism, configured to infuse insulin into the patient;
a program mechanism, configured to control the detection mechanism and the infusion mechanism, and at least based on the blood glucose value detected by the detection mechanism, the program mechanism generates an insulin infusion instruction and controls the infusion mechanism to perform the infusion, wherein:
the system adopts an infusion strategy consisting of pre-infusion and supplemental infusion, and an amount of insulin for pre-infusion is related to an estimated meal size at a time of pre-infusion, an amount of insulin for supplemental infusion is related to an estimated meal size at a time of supplemental infusion.

2. The closed-loop artificial pancreas system of claim 1, wherein,
the estimated meal size at the time of pre-infusion or supplemental infusion comprises different levels.

3. The closed-loop artificial pancreas system of claim 2, wherein,
the system determines whether to adjust the level of a next pre-infusion based on occurrence or absence of hyperglycemia and hypoglycemia within a predetermined time period after the pre-infusion.

4. The closed-loop artificial pancreas system of claim 3, wherein,
the level of next pre-infusion is not changed when the meal size of pre-infusion is at the highest level and hyperglycemia occurs within a predetermined time period after the pre-infusion, or when the meal size of pre-infusion is at the lowest level and hypoglycemia occurs within a predetermined time period after the pre-infusion.

5. The closed-loop artificial pancreas system of claim 2, wherein,
the system confirms whether to perform the supplemental infusion at least based on a blood glucose value after the pre-infusion has been completed for a predetermined period of time.

6. The closed-loop artificial pancreas system of claim 5, wherein,
the system determines whether to change the level of a next supplemental infusion based on occurrence or absence of hyperglycemia and hypoglycemia within a predetermined time period after the supplemental infusion.

7. The closed-loop artificial pancreas system of claim 6, wherein,
the next supplemental infusion level is not changed when the meal size of supplemental infusion is at the highest level and hyperglycemia occurs within a predetermined time period after the supplemental infusion, or when the meal size of supplemental infusion is at the lowest level and hypoglycemia occurs within a predetermined time period after the supplemental infusion.

8. The closed-loop artificial pancreas system of claim 1, wherein,
the amount of insulin for pre-infusion is related to an actual blood glucose value or its rate of change or IOB at a time of pre-infusion.

9. The closed-loop artificial pancreas system of claim 1, wherein,
the amount of insulin for supplemental infusion is related to an actual blood glucose value or its rate of change or IOB at a time of supplemental infusion.

10. The closed-loop artificial pancreas system of claim 2, wherein,
the program mechanism performs different meal mode function, the meal mode comprises a high carbohydrate mode and a normal carbohydrate mode.

11. The closed-loop artificial pancreas system of claim 10, wherein,
the high carbohydrate mode at least comprises a large mode and a regular mode, the normal carbohydrate mode at least comprises a regular mode.

12. The closed-loop artificial pancreas system of claim 11, wherein,
prior to the pre-infusion, if the user-selected meal mode changed, the system performs the pre-infusion or supplemental infusion based on a default estimated meal size.

13. The closed-loop artificial pancreas system of claim 12, wherein,
the default estimated meal size is any level of the different meal size levels.

14. The closed-loop artificial pancreas system of claim 13, wherein,
the default estimated meal size is the lowest level of the different meal size levels.

15. The closed-loop artificial pancreas system of claim 11 wherein,
the estimated meal size for the highest level of the regular mode is not greater than the estimated meal size for the lowest level of the large mode at the time of pre-infusion.

16. The closed-loop artificial pancreas system of claim 15, wherein,
the estimated meal size for the highest level of the regular mode is not greater than the estimated meal size for the lowest level of to the large mode at the time of supplemental infusion.

17. The closed-loop artificial pancreas system of claim 11, wherein,
the different levels of estimated meal size of pre-infusion are independent of that of supplemental infusion each other.

18. The closed-loop artificial pancreas system of claim 16, wherein,
the estimated meal size of pre-infusion is greater than that of supplemental infusion when they are at the same level.

19. The closed-loop artificial pancreas system of claim 11, wherein,
the high carbohydrate mode and/or the normal carbohydrate mode further comprise a small carbohydrate mode, the system performs insulin infusion based on a default estimated meal size in the small meal carbohydrate mode.
